# EUROPEAN PATENT APPLICATION

(11) **EP 4 129 351 A1**
(43) Date of publication of application: **08.02.2023**
(21) Application number: 21190007.1
(22) Date of filing: 05.08.2021
(51) Int. Cl.: A61L 31/02, A61L 31/08, A61L 31/14

(54) **METHOD FOR PRODUCTION OF NANOSTRUCTURED SURFACES ON STENTS FOR IMPROVED BIOCOMPATIBILITY**

(71) Applicant: Jozef Stefan Institute, 1000 Ljubljana (SI); Ürgen, Mustafa Kamil, Istanbul (TR)
(72) Inventor: ÜRGEN, Mustafa Kamil, Istanbul (TR); JUNKAR, Ita, 1000 Ljubljana (SI); KOVAC, Janez, 1000 Ljubljana (SI); RECEK, Nina, 1000 Ljubljana (SI); MOZETIC, Miran, 1000 Ljubljana (SI); YELKARASI, Çagatay, 34744 Istanbul (TR); KAZMANLI, Muhammet Kürsat, 34744 Istanbul (TR); KARAMAN, Berke, Istanbul (TR)
(74) Representative: Marton, Dan-Robert

(57) **Abstract**

The present invention relates to methods for suppressing unwanted effects upon installing a bare metallic stent into the human body, such as restenosis and thrombosis. A bare metallic stent made from a metal or an alloy of appropriate mechanical properties is subjected to a six-step procedure which includes selecting the appropriate metal or alloy, treatment with Ar ions, depositing a uniform film of titanium, anodization until a fraction of said titanium film is oxidized, removal of the said oxide film and activation of the product with atomic oxygen. The present invention also relates to a metallic stent made from a material of appropriate mechanical properties coated with a thin film of titanium with nanostructured surface and surface energy above 50 mN/m in accordance with the method of the present invention.

## Description

### FIELD OF THE INVENTION

The present invention relates to a method of producing metallic stents, in particular, bare metallic stent without drug-elution function.

### BACKGROUND OF THE INVENTION

Coronary stents are often applied for the treatment of coronary diseases. The stents are made from different materials, but most exhibit poor hemocompatibility, which is reflected in the increased risk of thrombosis. The patients are prescribed anti-thrombosis pills to suppress the risk of cloth formation, but the pills often have side-effects. The biggest issue with coronary stents, however, is the restenosis, i.e. uncontrolled growth of soft muscular cells. The restenosis stands between 20%-30% of the cases. During the procedure of stent implementation, the vessel wall is disrupted, which highly increases the risk of lumen narrowing or so-called stent. Restenosis is defined as 50% narrowing of vessels diameter and presents a serious concern in stenting. Moreover, it should be noted that stents can elicit allergic reactions most commonly those that contain Nickel, such as Nitinol and stainless steel.

The higher rates in restenosis occur in patients with high-risk factors, such as diabetes. To overcome this problem, the so-called drug-eluting stents (DES) have been developed, which should overcome this type of complications. It was actually shown that DES reduce the risk of restenosis as well as allergic reactions. Usually, DES release anti-cell-proliferative, immunosuppressive or anti-thrombogenic drugs which inhibit proliferation of smooth muscle cells and reduce thrombus formation. The main problem associated with DES is the inhibition of growth of normal endothelium, which presents a serious concern for stent-induced thrombosis. It was actually shown that thrombosis in the case of DES increases, which leads to stroke or heart attack with higher possibility. Thus, patients with DES stents should receive antiplatelet therapy thought their life.

Thus, novel approaches should be sought in order to improve surface interaction of metal stents and to prevent stent induced thrombosis as well as improve proliferation of natural antithrombogenic material based on natural endothelial cells. Companies manufacturing vascular stents are increasingly seeking for novel surface treatment procedures, but unfortunately, applications in this field have been only incremental, as mainly the research was focused on different types of polymer coatings and drug-eluting components, which are washed off after time and can also suppress proliferation of endothelial cells.

The success of stents is thus highly connected to the ability of the surface to prevent or avoid the aggregation of platelets in the blood vessels as well as to prevent uncontrolled proliferation of smooth muscle cells and simultaneouslyat the same time improve the proliferation of endothelial cells. Research in the field of nanomaterials has revealed that the nanotopography is one of the crucial factors influencing on the desired biological response. Thus, the fabrication of nanostructured chemically biocompatible materials could present the solution to the problem. Coating of metals with metal-oxide nanostructured films such as nanotubes or nanorods turned out a very efficient method for improving surface properties upon incubation with human blood. Thus, a very promising approach is based on coating the surface of the metallic stent with a thin layer of nanostructured titanium oxide like disclosed in US2010183501. The biocompatibility on the experimental scale was further improved by exposure of nanostructured titania films to oxygen plasma (cf. M. Kulkarni, A. et al., J. Nanomed. 10 (2015) 1359, I. Junkar, et al., J. of physics. D, Applied physics. 49 (2016) 24, I. Junkar et al., Could titanium dioxide nanotubes represent a viable support system for appropriate cells in vascular implants, A.Iglič, A.J. Garcia-Saez, M. Rappolt: Advances in biomembranes and lipid self-assembly vol. 25., Cambridge (MA), Academic Press, cop. 2017. p. 1-39). However, the mechanical stability of nanotubes is poor and the structures can easily be detached from the surface upon mechanical stress. This further leads to high risk of cracking and/or peeling off the nanostructured surface film, which ruins the desired surface morphology and desired biocompatibility. Moreover, complications with the release of nanotubes in the biological environment may cause severe problems like infections and allergic reactions. In an ideal case, a coating on a bare metallic stent is as flexible as the metallic substrate itself but as anti-thrombogenic as a plasma-treated nanostructured titanium oxide film.

Hence, nanostructured morphology based on stable titanium oxide layer was revealed in US2010183501, for instance. Therein a titanium layer is formed on the stent surface, and the titanium layer is nanostructured in the form of titanium oxide nanotubes. The titanium is deposited on the medical implant, and by electrochemical anodization, the nanotubes with different length and diameters are formed.

Moreover, nanotubular surfaces are fabricated by electrochemical anodization process in US20060229715. The surface consists of an oxide metal layer which has nanotubes with pore diameter between 15 to 100 nm and height of the tube between 15 to 5000 nm. Furthermore, the nanotubes are annealed at a temperature between 280 and about 580° C. The EP3459569 A1 patent discloses the invention, where Ti or Ti alloy substrates are used for the growth of nanotubes by an electrochemical anodization process. Afterwards, the nanotubes are removed from the surface by sonification in H₂O₂ or organic acid or base aqueous solution. The surface exhibits nanostructures of diameter between 10 to 1.000 nm. In this invention no further treatment to improve surface properties is presented, nor is the coating of non-Ti-based substrates with titanium.

US20110236435 discloses a method of growing bone cells on TiO2 nanotubular substrates treated by plasma. In this case the titanium substrate is anodized to form TiO₂ nanotubular array which is afterwards subjected to a radiofrequency plasma discharge to chemically modify the array and to seed bone cells in order to improve their growth. The RF discharge is used for the treatment of nanotubes with several gases: nitrogen, oxygen, a mixture of nitrogen and oxygen and helium. The size of the nanotubes inner diameter is from about 80 to 107 nm. Optimal results were obtained with plasma created in a mixture of nitrogen and oxygen. The functional groups provide a suitable surface finish for greater fibrinogen adsorption resulting in enhanced cell growth. The treatment was shown to improve the growth of bone cells, but is due to increased fibrinogen adsorption inappropriate for interaction with blood, as it is known that fibrinogen adsorption promotes platelet activation and adhesion.

In WO2018029166, the combination of fabrication of titanium oxide nanotubes and highly reactive oxygen species is disclosed. By this method reduction of platelet adhesion and activation on the surface is achieved.

Many other methods have been disclosed to reduce the risk of restenosis. Most of them are dealing with the development of drug-delivery stents. They minimize the risk of thrombosis, but since they inhibit endothelialization in the long term, their risk of restenosis is higher compared to the coated stents.

### Problem to be solved

Thus, one problem to be solved according to the present invention is supressing the adhesion of platelets and smooth muscle cells on the medical devices and simultaneous provision of optimal conditions for the growth of endothelial cells.

### Solution according to the invention

The solution of this problem is achieved by means of a method in accordance with claim 1 and a metallic device to be implanted, preferably metallic stent or vascular stent according to claim 8.

### SUMMARY OF THE INVENTION

The present invention is defined by the appended independent claims. Preferred embodiments of the invention are defined by the dependent claims.

The present invention hence relates to metallic stents which are selected in a first step (Step #1), such as Cobalt-Chromium (CoCr), titanium alloys, Nitinol (NiTi alloy) and stainless steel. In the presented example, the stainless steel (316LVM, medical-grade) substrates can be first exposed according to an embodiment to a fluence of energetic argon ions under high-vacuum conditions (Step #2). But generally, other metallic substrates can be used within the scope of the present invention. This treatment causes removal of any impurities that are likely to be present on the surface of alloys, such as traces of organic impurities, native oxides and any segregation of alloying elements. According to the methods of invention, the treatment by argon ions also causes high surface energy of the material, that is due to the absence of any impurities. The surface morphology after the treatment with the argon ions remains almost unchanged, very smooth. However, a proper adhesion between the coating and the stent can be ensured by the aforementioned treatment step, which is an ion-etching cleaning process step, especially performed by means of Ar ions as a possible embodiment.

The third step (Step #3) according to the method of the present invention is a deposition of a titanium film. The titanium film is deposited onto the stainless steel substrates or other similar metallic medical materials which had been pretreated in Step #2 without breaking the vacuum conditions. After accomplishing the Step #2, the substrates are subjected to super-thermal titanium atoms of average kinetic energy in the range of few to tenths of eV by using a suitable PVD method such as DC, pulse, RF, HiPIMS, magnetron sputtering using planar, circular or rotating targets or cathodic arc. This step (Step #3) is provided in order to obtain a structured titanium nano-matrix on the substrate, which will be subsequently processed according to the present invention.

However, a further suitable PVD method in Step #3 can be e-beam evaporation and/or laser ablation. It may also be conceivable in the scope of the present invention that a combination of the at least above-mentioned PVD methods can be used for deposition of the titanium film.

The titanium atoms condense on the stainless-steel substrates and form a thin homogeneous film with a thickness in the range of 200 nm to 10000 nm. In preferred embodiments, the thickness of the titanium film is between 500 nm - 2000 nm.

The fourth step (Step #4) is partial oxidation of the titanium film which has been deposited according to Step #3 onto the stainless steel or other similar substrates pretreated according to Step #2. The oxidation is preferably performed by electrochemical anodization. The electrochemical anodization is accomplished by immersion of the substrates prepared according to Steps # 2-3 in an electrolyte that preferably contains ethylene glycol, water and NH₄F at temperatures between 0 °C and 80°C. Other halide-ions (such as NH₄F, HF of HCI) containing compounds and organic/water-based electrolytes (such as glycerol) can also be used for the same purpose. The DC voltage applied to substrates prepared according to Steps # 2-3 is in the range of 5 - 150 V, preferably 30 - 70 V. The treatment of Step #4 results in partial oxidation of the titanium film on the stainless steel surface, so a porous film of titanium oxide (titania) appears on the surface of the titanium-coated stainless steel. The thickness of the oxide film can be tuned by anodization parameters such as anodization time, voltage and temperature. The oxide film thickness can be in the range of 5 - 90% of the deposited titanium film thickness. In the preferred embodiment, the thickness of remnant metallic titanium film below the titanium anodic oxide is in the range of 100 nm to 2000 nm. The film of titanium oxide synthesized according to the invention is preferably in the form of titania nanotubes. The titania nanotubes are dense and preferably of almost the same diameter. The structure of material after accomplishing the Step #4 is, therefore, smooth stainless steel (stainless steel as an example) substrate covered with a thin film of pure titanium that has a periodically varied thickness in lateral dimensions which is additionally covered with titania nanotubes.

In the fifth step (Step #5) the titania nanotubes are removed without causing significant modification on the metallic titanium layer or the rest of the structure that has not been oxidized in Step #4. The removal of the titania nanotubes is preferably, but not limited hereto, accomplished by ultrasonication in distilled water or by other mechanical means such as peeling them off by using adhesive tapes. The ultrasonication (Step #5) according to one possible embodiment enables complete removal of the oxide film on the substrate prepared according to Steps #2-4 without causing a significant modification of the metallic component of the stent. The morphology of the metallic component is preserved upon treatment according to Step #5. The titania film assumed a rich morphology upon treatment of Step #4 that leads to the nanopatterning of the titanium coating below it. Thus by the removal of weakly attached top oxide layer performed in Step #5, a nanostructured metallic titanium surface is attained. The thickness of the metallic film that has remained after Step #5 is preferably in the range of 100 - 2000 nm. Therefore, Step #5 results in a nanostructure rich morphology of the remaining titanium film that was found beneficial. This step is crucial since it provides circular morphology on the surface and enables only a thin layer of titanium oxide on the surface, which results in mechanically much more stable coating.

Thus, the surface obtained according to the present invention corresponds to a thin metallic titanium film of nanostructured surface in the form of dense nano-sized interconnected pores of pore depth of at least 5 nm and diameter less than 160 nm for instance, according to the present invention, advantageously a distance between neighbouring pores less than 30 nm is provided.

The stents produced according to Steps #2 - 5 of the present invention are further treated in a final Step #6. Step #6 employs a brief treatment with oxygen plasma. This treatment assures optimal surface energy and chemistry. The combination of surface modification techniques on stainless steel or other similar surfaces a titanium film of rich morphology and appropriate chemistry are fabricated which suppress proliferation of smooth muscle cells and enhance the proliferation of endothelial cells, while at the same time prevent adhesion and activation of platelets on the surface.

### EMBODIMENTS OF THE INVENTION

According to an embodiment said bare metallic stent is made from a material comprising Cobalt-Chromium (CoCr), titanium alloys, Nitinol (NiTi alloy) and/or stainless steel (316L, medical-grade). These materials have shown very good material characteristics to be treated by the method according to the present invention.

According to an embodiment said depositing of the said uniform film of metallic titanium on of the above mentioned materials leads to a thickness between 200 and 10000 nm of a said uniform film, whereby a PVD method is used. The PVD method ensured to achieve the above-mentioned thickness, which was the best range for the further treatment steps according to the present invention.

According to an embodiment said anodic oxidation is performed in halide-ion containing ethylene-glycol as electrolytes at temperatures between 0 °C and 80 °C, with water content between 0.5 to 10 vol% within the voltage range of 5 - 150 V, preferably 30 - 70 V, and halide-ion concentration between 0.1 to 5 vol% for a time duration between 5 s to 3 h. The inventor figured out that the above-mentioned anodization step characteristics provided the best results according to the present invention.

According to an embodiment removal of the said anodic oxide film is performed by ultrasonication. It was observed that ultrasonication caused a non-significant modification of the metallic component of the stent.

According to an embodiment said exposure to atomic oxygen is performed in an oxygen plasma of O-atom density between 1x10¹⁹ and 1x10²² m⁻³ and oxygen ion density below 10¹⁷ m⁻³. This plasma treatment of the metallic stent is to be performed prior interaction with the blood of a patient. After this step, the device is ready to be implanted and showed best mode features for later usage in an hospital for instance.

According to a further object of the present invention, a metallic device to be implanted, preferably metallic stent, is provided. The device comprises a material coated with a thin metallic titanium film of nanostructured surface. The nanostructured surface of metallic titanium is in the form of nano-sized interconnected pores with widths between 15 and 160 nm, preferably between 40 and 120 nm and said nanostructured metallic titanium film of a thickness of 100 - 2000 nm, preferably 200 - 1000 nm. The interconnected pores are in the form of hemispherical holes and have depths between 5 and 150 nm, preferably between 10 and 30 nm. According to the invention, the above-mentioned properties showed best results during usage of the metallic stent in accordance with the invention.

According to an embodiment said surface free-energy is achieved by treatment with atomic oxygen in oxygen-containing gaseous plasma, wherein said treatment is performed prior to blood interaction of said device. Thus the device, according to the invention, is ready to be implanted.

Hence, the obtained surface morphology substantially differs from the already known nano-tubes matrixes on medical devices. Furthermore, mechanical stability and desired surface morphology are provided by means of the present invention.

Unlike state of the art, the bare metallic stent will be coated according to the present invention with a titanium film free from a fragile oxide coating but of appropriate surface morphology and appropriate surface energy. Such stents are useful to prevent post-implantation complications such as restenosis and thrombosis and stimulate proliferation of natural vascular endothelial cells.

Hence, the fabrication of stable nanostructured surfaces with appropriate mechanical stability which assures the implantable device to function in the body is provided.

The accompanying figures are included to provide a further understanding of the present invention and are incorporated in and constitute a part of this specification. The drawings illustrate the embodiments of the present invention and together with the description, serve to explain the principles of the invention. Other embodiments of the present invention and many of the intended advantages of the present invention will be readily appreciated as they become better understood by reference to the following detailed description.

### BRIEF DESCRIPTION OF THE DRAWINGS

The invention is explained in further detail, by way of example and with reference to the accompanying drawings wherein:
- Fig. 1: shows a schematic of the 6-step procedure in accordance with the present invention;

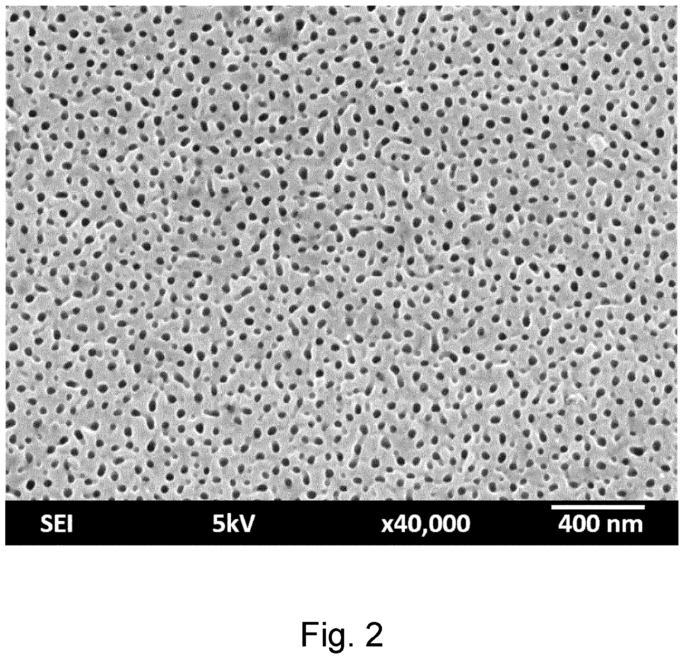

- Fig. 2: shows a SEM image of the product after accomplishing the anodization so that the titania film of rich morphology (nanotubular structure) with an average thickness of 500 nm is formed on the metallic titanium surface.;

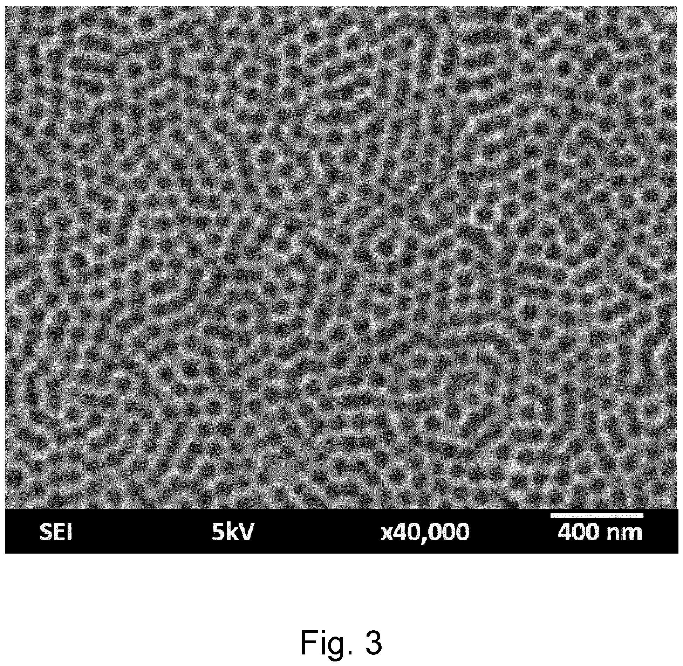

- Fig. 3: shows a SEM image of the product after accomplishing the ultrasonication so that the anodically oxidized titania film of rich morphology is removed from the surface and the non-oxidized segment of titanium film assumes a rich nanostructured morphology;
- Fig. 4: shows a SEM image of a standard vascular stent coated with anodically oxidized titania film after accomplishing bending tests. The oxide film cracks upon mechanical forces;

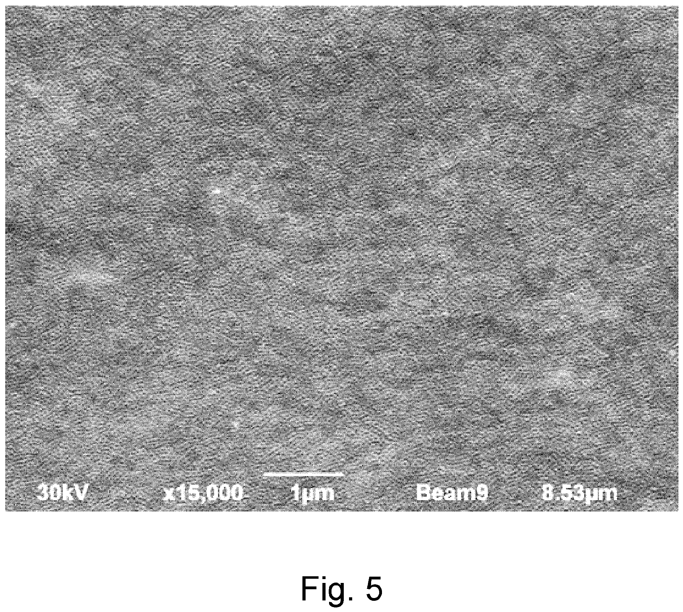

- Fig. 5: shows a SEM image of an innovative vascular stent produced by removal of the anodically oxidized titania layer after the accomplishment of the bending tests. The product remains intact - no cracks are observed;
- Fig. 6: shows an AFM image of the surface an innovative stent after accomplishing all six steps according to Figure 1;
- Fig. 7: shows SEM image of the product synthesized according to the 6-step method shown schematically in Figure 1 (a-e) after incubation with whole human blood. No platelets were detected on the surface-modified according to the present invention;
- Fig. 8: shows SEM image of a titanium surface after incubation with whole human blood, where platelets are mainly in the fully-spread form and thus highly activated on the surface, wherein similar observations are provided for stainless steel surfaces;
- Fig. 9: shows *in vitro* biological response of endothelial cells on oxygen plasma treated plain titanium (pTi) and plasma modified nanostructured titanium surface (surface modified according to the present invention) (cTi), normalized to stainless steel surface used as control; and
- Fig. 10: shows *In vitro* biological response of smooth muscle cells on plain titanium plasma modified surface (pTi) and plasma modified titanium oxide nanostructured surface of circular morphology (cTi) (surface modified according to present invention) normalized to stainless steel surface used as control.

### DETAILED DESCRIPTION OF THE INVENTION

The present invention relates to methods for optimization of the surface finish of vascular stents. Without wishing to be bound by theory, it is assumed that both the surface morphology and surface energy influence the adsorption and conformation of blood proteins, activation of blood platelets, cloth formation, and proliferation of endothelial and smooth muscle cells.

To achieve a desired biological response, the attachment of proteins and cells to the surface of biomaterials is of primary importance. When a biomaterial is exposed to a biological environment, many extremely complex reactions may occur at the cell-biomaterial interface. These reactions include coagulation, healing, inflammation, mutagenicity etc.

Immediately after the contact of a biomaterial surface with body fluids, the layer of proteins is adsorbed in the so-called "race for the surface", which further dictates interactions with cells. Cells may strongly adhere to some types of surfaces while they will not adhere to others. The main reason lies in the special structure of individual cell membranes and the surface properties of biomaterial and cell-specific interaction with proteins. As the proteins are the first to reach the biomaterial surface, the cells actually interact with a biomaterial surface covered by the protein layer. Thus, it is of primary importance to condition the surface for desired protein adsorption. The protein adsorption is an interfacial phenomenon and is strongly dependent on the physicochemical properties of the biomaterial surface such as surface energy, surface charge, (nano) topography etc. It was observed that the type, the amount and the conformational state of the adsorbed proteins dictate platelets adhesion and activation. Surface wettability is believed to highly influence the amount of adsorbed proteins as well as on their conformational state, which further dictates cell adhesion. Generally, hydrophobic surfaces are considered to be more protein-adsorbent than hydrophilic surfaces, due to strong hydrophobic interactions occurring at these surfaces. For example, adsorption of fibrinogen (Fg), a protein which is present in blood plasma, has been shown to be closely related to surface-induced thrombosis. Consequently, strategies for preventing/reducing fibrinogen adhesion on surfaces interacting with whole blood have been developed. It was already shown that fibrinogen molecule, will not adhere to hydrophilic negatively charged surfaces, which further reduces the risk of platelet adhesion and activation on these surfaces. Adhesion and platelet activation degree can be studied from the shape and number of platelets interacting with the biomaterial surface. The cells in more round form present the non-activated state, while dendritic or more spread morphology will correspond to its activated state, which will with high possibility lead to undesired thrombotic reactions, fibrin and clot formation (thrombosis).

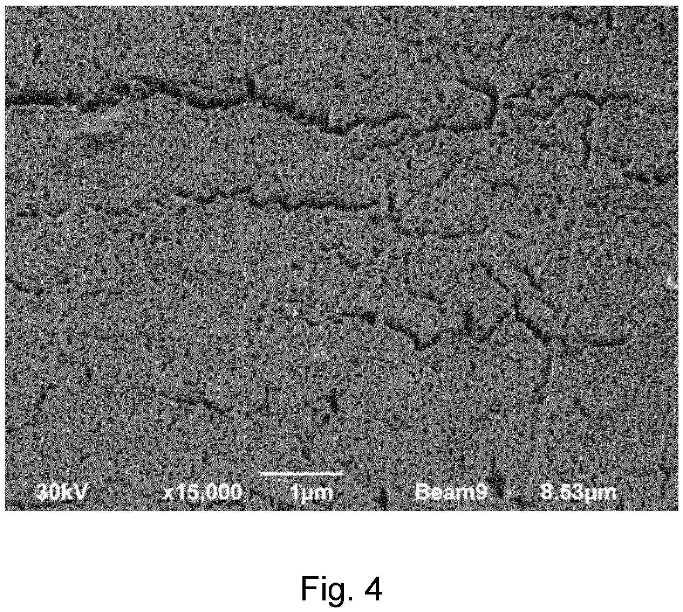

The transformation of fibrinogen to fibrin fibres is stimulated by enzymes and factors released from blood platelets upon interaction with a surface. The interaction will occur on smooth surfaces since the contact area between a platelet and a smooth surface is large. In cases of nanostructured surfaces of appropriate aspect ratio, the contact area is minimized, and so is the activation of blood platelets. The optimal surface finish for suppressing cloth formation is, therefore nanostructured and hydrophilic surface. Titania nanotubes activated by a brief treatment with oxygen plasma satisfy this condition. The drawback of activated titania nanotubes is, however, poor mechanical properties. Figure 4 shows a SEM image of a stent coated with titania nanotubes after performing the bending tests. The coating of nanotubes cracks. This effect is regarded as detrimental for the application of vascular stents coated with titania nanotubes.

The optimal condition will be thus the surface morphology similar to titania nanotubes, but free from the fragile coating. The methods for achieving the optimal conditions are disclosed herein. In the preferred embodiment, the titania nanotubes serve as a template for a nanostructured titanium surface, which is further activated by a brief treatment with oxygen plasma.

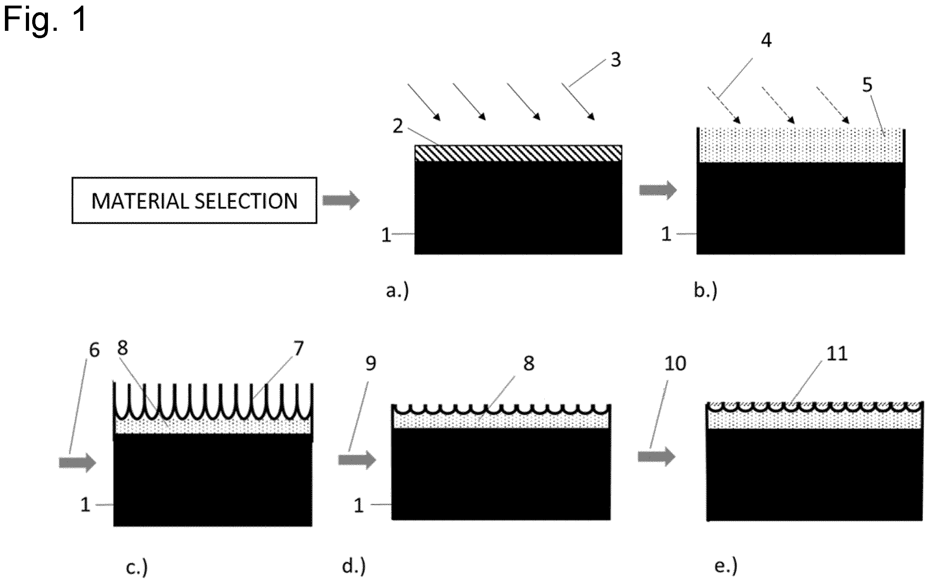

The methods of invention reveal the procedure performed in order to obtain the optimal conditions. The bare stainless steel vascular stents 1 are smooth as shown in Figure 1 (a). The bare stents 1 are always covered with a contamination layer 2. The contamination layer 2 makes the bare stent 1 hydrophobic, so adhesion of any metallic coating is inappropriate. The surface energy is increased by the removal of the thin film of contamination layer 2. In preferred embodiments, the thin film of contamination layer 2 is removed by exposure of the bare stent 1 to pre-clearing procedure 3, where argon ions are used. The argon ions cause sputtering of material on the surface of the bare stent 1. The appropriate kinetic energy and fluence of the argon ions 3 on the surface of a bare stent 1 with a contamination layer 2 will cause the removal of the contamination layer 2 and thus optimal surface energy of the bare stent 1 is obtained. This is Step #2 of the methods of the invention and is schematically presented in Figure 1 (a). The ion energy and the fluence can be in a broad range of parameters, but preferably the ion energy is between 500 and 3000 eV, and the fluence between 10²⁴ and 10²⁶ m⁻². The temperature of the bare stent 1 upon treatment shown in Figure 1 (a) is below 500 °C, preferably between 25 and 200 °C.

The bare stent 1 treated by argon ions 3 is then exposed to super-thermal titanium atoms 4. In preferred embodiments, the source of titanium atoms 4 is a dual rotating titanium target treated in a magnetron discharge. The discharge enables sputtering and thus release of the titanium atoms 4 which condensed on the surface of bare stent 1 to form a film of pure titanium 5 (Step #3). A variety of thicknesses for deposited titanium film 5 have been found useful. In a preferred embodiment, the average thickness of the deposited titanium film 5 is between 200 nm and 10000 nm, most preferably between 500 and 2000 nm. The adhesion between the bare stent 1 and the titanium film 5 is optimal due to prior treatment of the bare stent with the argon ions 3. The surface of bare stent 1 after depositing the titanium film 5 remains smooth. As explained above, the smooth surfaces are not beneficial due to rapid activation of blood platelets. Step #3 of the methods of invention is presented schematically in Figure 1 (b).

According to the present invention, the rich morphology of the titanium film is achieved by two further treatments, i.e., first the selective and incomplete oxidation (step #4), followed by removal of the oxide layer (step #5). A suitable technique for the formation of an oxide film of extremely rich morphology is anodization. The anodization is a process comprising immersing a product into an electrically conductive solution and applying a positive voltage onto the product. The oxidation propagates in a controlled manner providing the anodization parameters are kept within the prescribed ranges.

In a preferred embodiment, the anodization (6 in Figure 1) is performed in a solution that contains an organic liquid, water and a source of ions that catalyze oxidation. In a further preferred embodiment, the solution contains ethylene glycol, water and ammonium fluoride. Ethylene glycol serves as the organic liquid, water as a source of oxygen and ammonium fluoride as a catalyzer. The concentration of the source of oxygen and catalyzer is preferably below 10% to keep the oxidation process in a controlled manner. In a most preferred embodiment, the concentration of water and ammonium fluoride is about 2 vol% and 0.6 vol%, respectively. It shall be understood that the concentration can be different from the most preferred embodiment. The ions in the solution will cause significant oxidation of the product immersed in the solution by anodically polarizing. The stent is thus connected to a DC power supply and anodically polarized against the liquid solution. The oxidation proceeds according to the applied voltage. According to the present invention, the voltage is between 5 and 150 V, in preferred embodiments between 30 and 70 V. The most preferred voltage is 40 V. A person with skills will know that the preferred applied voltage depends not only on the composition of the liquid electrolyte but also to dimensions, temperature etc. It shall be understood that the applied voltage can be different from the most preferred embodiment. The temperature range for this step (Step #4) is between 0 °C and 80°C.

The anodization 6 will lead to the formation of titania nanotubes 7 as presented schematically in Figure 1 (c). The interface between the stainless steel stent 1 and the titanium film 5 remains smooth after accomplishing the anodization. The interface with the oxide film, however, assumes periodically modulated structure. The non-oxidized fraction of the titanium film 8, therefore assumes periodically modulated thickness as shown schematically in Figure 1 (c). A variety of thicknesses for the non-oxidized fraction of the titanium film 8 have been found useful. In a preferred embodiment, the average thickness of the remnant (non-oxidized) titanium film 8 is between 100 nm and 2000 nm, most preferably between 200 and 1000 nm. It shall be understood that the thickness of the remnant (non-oxidized) titanium film 8 can be different from the most preferred embodiment. The periodically modulated thickness of the titanium film 8 follows the structure of the titania nanotubes 7. The morphology of the periodically modulated thickness of the titanium film 8 is tailored by changing the parameters of anodization 6.

Step #5 of the present invention is shown schematically in Figure 1 (d). The product shown in Figure 1 (c) is subjected to ultrasonication 9, for instance, but other procedures are conceivable within the scope of the present invention. The ultrasonication causes removal of the titania film 7 from the surface of the product. The ultrasonication can be performed in a broad range of parameters. In the preferred embodiment, the peak-to-peak amplitude of the transversal mechanical oscillations is between 10 and 100 µm, most preferably between 20 and 30 µm. The oscillation frequency is preferable between 15 and 50 kHz, most preferably between 18 and 25 kHz. A preferred ultrasonication treatment time is between 10 and 600 s, most preferably between 40 and 100 s. Such a treatment time enables removal of the entire titania coating 7 from the surface of the non-oxidized fraction of the titanium film 8. It shall be understood that the ultrasonication parameters can be different from the most preferred embodiment. The final surface finish after completing the steps #2-5 according to the present invention is shown in Figure 1 (d). The oxygen plasma treatment 10 (Step #6) enables activation of the surface of the non-oxidized fraction of the titanium film 8 with polar groups 11 and thus high hydrophilicity. The plasma treatment 10 may be performed in any oxygen-containing gas using any electrical discharge. In one experiment, the treatment was performed in oxygen plasma sustained by a high-frequency electrical discharge of power density about 30 W per litre, and for 10 s. the treatment in oxygen plasma 10 enables both surface functionalization 11 and formation of a very thin oxide film on the surface of the non-oxidized fraction of the titanium film 8, as shown schematically in Figure 1 (e). In preferred embodiments, the thickness of the very thin oxide film on the surface of the non-oxidized fraction of the titanium film 8 is about 5 to 10 nm. Such a thickness is beneficial for chemical inertness of the product as well as excellent hydrophilicity, and on the other hand, it is thin enough to prevent cracking even upon severe conditions. It shall be understood that the applied voltage can be different from the most preferred embodiment.

Hence, the methodology steps according to the present invention schematically shown with reference to Fig. 1 are summarized in the following. The smooth stainless steel substrate 1 is covered with a contamination layer 2. The surface is subjected to argon ions 3 to remove the contamination layer 2 and cause optimal surface energy of the stainless steel substrate 1. Without breaking the vacuum conditions, the stainless steel substrate is subjected to a random flux of low-energy titanium atoms/ions 4, which condense on the surface of stainless steel substrate 1 causing the formation of a rather uniform film of titanium 5. The product is then immersed into anodization bath 6 which causes the formation of a porous titania film 7. The oxidation occurs in such a way that the non-oxidized segment of titanium film 8 assumes a rich morphology. The product is then transferred to ultrasound bath 9, which causes removal of the nanoporous titania film 7. The product is then exposed to oxygen plasma 10 so that the surface of remnant nanopatterned metallic titanium film 8 is enriched with polar groups 11.

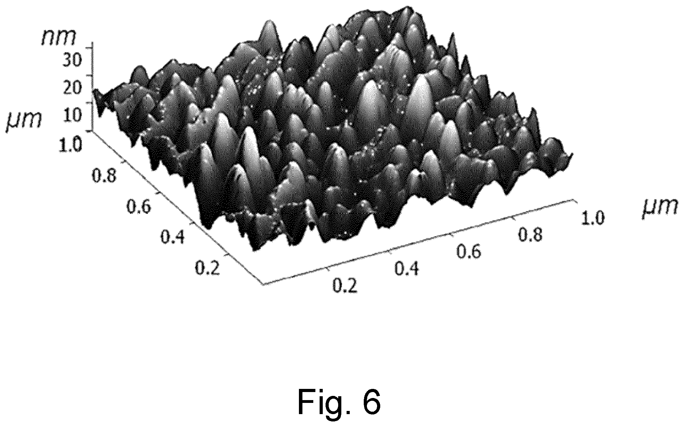

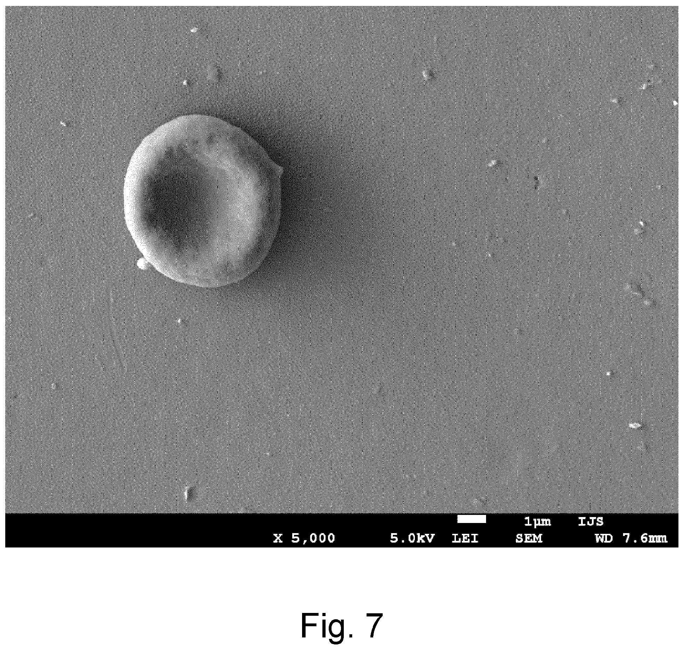

Figure 6 shows an AFM image of the surface of an innovative stent after accomplishing all the steps, according to Figure 1 (a-e). There are dense conical structures stretching from the surface of the product synthesized according to the Steps #2-5, shown schematically in Figure 1 (a-d). Such a surface morphology, together with polar groups 11, enables hydrophilic surface finish (Figure 1 (e)). A water droplet placed onto the surface of the product synthesized according to the 6-step method shown schematically in Figure 1 (a-e) spreads on the entire surface including the gaps between the cones due to capillary effect. Such a surface finish is beneficial since it prevents the accumulation of blood proteins on the surface as well as activation of blood platelets. A SEM image of the product synthesized according to the 6-step method shown schematically in Figure 1 (a-e) after incubation with human blood is shown in Figure 7. There are no platelets on the surface of the product synthesized according to the preferred embodiment of the innovative method.

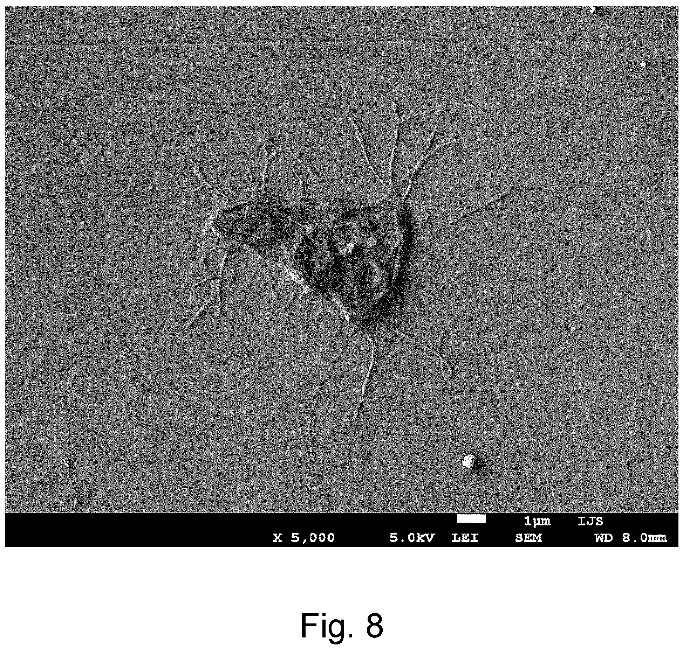

For comparison, Figure 8 shows the SEM image of a smooth titanium surface after incubation with whole human blood. Platelets are activated (spread) on the surface. The image presents the initial step that may lead to the formation of cloth on the surface of such a titanium surface.

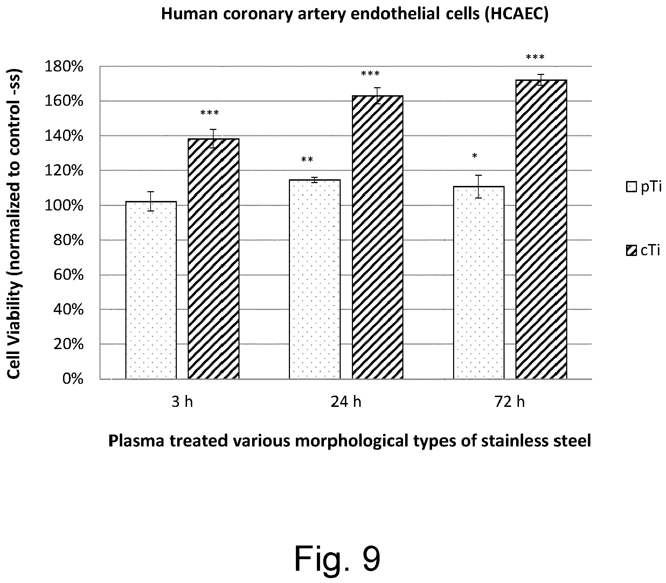

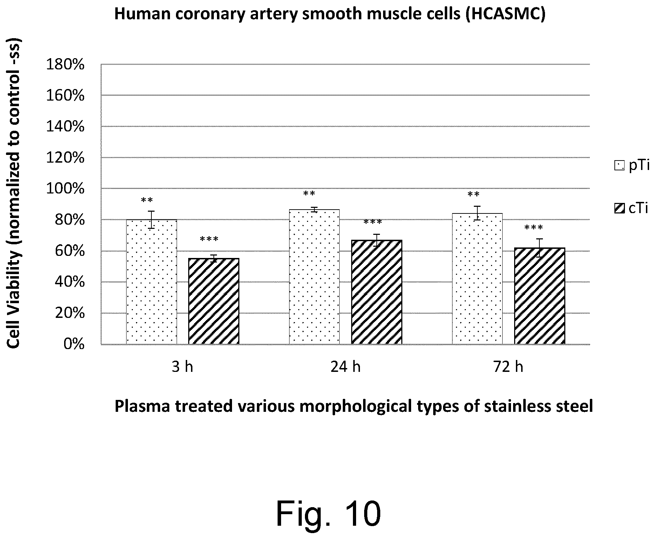

Figure 9 presents the viability of endothelial cells interacting with plain titanium surface (pTi) after plasma treatment and titanium surface fabricated by steps #2-6 with rich surface morphology and chemistry (cTi). Results are normalized to control stainless steel (SS) surface. Viability of endothelial cells significantly increases on titanium surface after the application of the method of invention shown in Fig 1 (a-e). The control SS surface exhibits the lowest viability of endothelial cells, followed by the plain titanium surface after treatment with only Step #6 according to the present invention and the highest viability is observed in case of surface fabricated by the steps disclosed in the present invention (#2-6). Thus, all the disclosed steps are crucial for achieving optimal biological response in terms of endothelial cell proliferation and as shown in Figure 10 also reduced smooth muscle cell adhesion- viability.

Figure 10 presents the viability of smooth muscle cells, which should be suppressed on the surface of blood connecting devices. The results indicate that viability of smooth muscle cells was significantly reduced on the surface fabricated by the steps disclosed in the present invention (#2-6) (cTi), while the highest viability was observed on the SS control sample followed by the plain titanium surface (pTi) after treatment only by step #6 of the present invention.

It is to be noticed that the term "comprising", used in the claims, should not be interpreted as being restricted to the means listed thereafter; it does not exclude other elements or steps. Thus, it is to be interpreted as specifying the presence of the stated features, integers, steps or components as referred to, but does not preclude the presence or addition of one or more other features.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Although specific embodiments have been illustrated and described herein, it will be appreciated by those of ordinary skill in the art that a variety of alternate and/or equivalent implementations may be substituted for the specific embodiments illustrated and described without departing from the scope of the present invention. This application is intended to cover any adaptations or variations of the specific embodiments discussed herein. Therefore, it is intended that this invention be limited only by the claims and the equivalents thereof.

## Claims

1. A method for producing a metallic stent, comprising:
(1) selecting a bare metallic stent;
(2) treatment of said bare metallic for cleaning purposes, especially by means of ion etching, especially with Ar ions of energy between 100 and 10000 eV upon vacuum conditions using a partial pressure of Ar in the range 0.2-4 Pa;
(3) depositing a uniform film of metallic titanium;
(4) anodization of said bare metallic stent coated with said uniform metallic titanium film until a fraction of 5 - 90% of said uniform titanium film is oxidized to an oxide film;
(5) removal of said oxide film from said bare metallic stent coated with said titanium film;
(6) exposure of said stent treated according to the preceding steps to atomic oxygen to obtain said metallic stent.

2. A method according to claim 1, wherein said bare metallic stent is made from a material comprising Cobalt-Chromium (CoCr), titanium alloys, Nitinol (NiTi alloy) and/or stainless steel (316L, medical-grade).

3. A method according to claim 1 or 2, wherein said depositing of the said uniform film of metallic titanium leads to a thickness between 200 and 10000 nm of a said uniform film, whereby a PVD method is used.

4. A method according to at least one of the preceding claims, wherein said exposure to oxygen atoms is provided at a fluence between 1x10²¹ and 1x10²⁵ m⁻², preferably between 1x10²² and 1x10²⁴ m⁻².

5. A method according to at least one of the preceding claims, wherein said anodization is performed in halide-ion containing electrolytes at temperatures between 0 °C and 80 °C, with water content between 0.5 to 10 vol% within the potential range of 5 - 150 V, preferably 30 - 70 V, and halide-ion concentration between 0.1 to 5 vol% for a time duration between 5 s and 3 h.

6. A method according to at least one of the preceding claims, wherein removal of the said oxide film is performed by ultrasonication.

7. A method according to at least one of the preceding claims, wherein said exposure to atomic oxygen is performed in an oxygen plasma of O-atom density between 1x10¹⁹ and 1x10²² m⁻³ and oxygen ion density below 10¹⁷ m⁻³.

8. A metallic device to be implanted, preferably metallic stent, comprising a material coated with a thin metallic titanium film of nanostructured surface in the form of dense nano-sized interconnected pores of pore depth between 5 nm and 150 nm, and diameter between 15 nm and 160 nm, said titanium film provided according to a method according to at least one of claims 1 to 7.

9. A device according to claim 8, wherein said material is selected from the list of materials with suitable properties including but not limited to Cobalt-Chromium (CoCr), titanium alloys, Nitinol (NiTi alloy) and stainless steel (316L, medical-grade).

10. A device according to claim 8, wherein the average thickness of the titanium film is between 100 and 2000 nm, preferably between 200 and 1000 nm.

11. A device according to claim 8, wherein said nanostructured surface of metallic titanium is in the form of nano-sized interconnected pores with widths between 15 and 160 nm, preferably between 40 and 120 nm.

12. A device according to claim 8, wherein said metallic titanium film of a thickness of 100 - 2000 nm, preferably 200 - 1000 nm, is structured with the said nanostructured surface in the form of hemispherical holes of diameter between 15 and 160 nm, preferably between 40 and 120 nm, and depths between 5 and 150 nm, preferably between 10 and 30 nm.

13. A device according to any of the claims 8 to 12, wherein surface free-energy is above 50 mN/m preferably above 60 mN/m, most preferably between 60 to 70 mN/m.

14. A device according to claim 13, wherein said surface free-energy is achieved by treatment with atomic oxygen in an oxygen-containing gaseous plasma, wherein said treatment is performed prior to blood interaction of said device.

15. Use of a device of claims 8 to 14 in medicine for treatment of vascular diseases.
